# EUROPEAN PATENT APPLICATION

(11) **EP 4 130 025 A1**
(43) Date of publication of application: **08.02.2023**
(21) Application number: 21775333.4
(22) Date of filing: 22.03.2021
(51) Int. Cl.: C07K 14/47, C07K 7/06, A61K 38/08, A61K 38/10, A61P 35/00

(54) **COMPOSITION COMPRISING VGLL1 PEPTIDE FOR TREATMENT OF CANCER**

(30) Priority: 27.03.2020 KR 20200037335
(71) Applicant: Onecuregen Co., Ltd., Daejeon 34141 (KR)
(72) Inventor: WON, Mi Sun, Daejeon 34141 (KR); KIM, Bo Kyung, Daejeon 34141 (KR); IM, Joo-Young, Daejeon 34141 (KR); KIM, Da Mi, Daejeon 34141 (KR)
(74) Representative: Viering, Jentschura & Partner mbB Patent- und Rechtsanwälte
(86) International application number: PCT/KR2021/003496
(87) International publication number: WO 2021/194186

(57) **Abstract**

The present invention relates to a peptide fragment for treatment of cancer, a variant thereof, and a use thereof. A peptide fragment of VGLL1 and/or a variant thereof according to the present invention inhibits the proliferation and invasion of cancer cells, thereby exhibiting an excellent effect on the prevention and treatment of cancer.

## Description

### [Technical Field]

The present invention relates to a composition for treating cancer, the composition including a VGLL1 peptide and a method for treating cancer using the same.

The present invention was made with the support of the Korean government in accordance with the Ministry of Science and ICT's task number 1711098598, "Basic Research in Science and Engineering."

The present invention was made with the support of the Korean government in accordance with the Ministry of Science and ICT's task number 1711081422, "Original technology development project (biomedical technology)."

The present invention was made with the support of the Korean government according to the Ministry of Science and ICT's task number 1711100103, "Main Project (2019-2023)."

### [Background Art]

Cancer has a high mortality rate worldwide and is the second most common cause of death in Western societies after cardiovascular disease. In particular, colorectal cancer, breast cancer, prostate cancer, etc., are continuously increasing due to the aging of the population, generalization of high-fat diet intake due to westernization of diet, rapid increase in environmental pollutants, and increase in alcohol consumption. In this situation, there is an urgent need for the creation of anticancer substances that can contribute to the promotion of human health, the improvement of the quality of a healthy life, and the promotion of human health by enabling the early prevention and treatment of cancer.

The biggest factor in the development of cancer is the occurrence of mutations in genes. A substance that causes mutation is called a carcinogen, and the step in which genetic modification occurs by this carcinogen is called initiation. Among the genes included in the initiation step, there are carcinogen suppressor genes (e.g., p53, PTEN, INK family, RB) and constitutive active mutations (active mutants, such as EGFR^{mt}, RasG^{12V}) that are always active due to mutation. In normal cells, the epidermal growth factor binds to the EGF receptor to create an activation signal, but active mutants always send an activation signal to the downstream signaling system even if the ligand does not bind to the receptor. One mutant cell generated in this process must break through the immune defense system in the body to grow into a tumor mass. This process is called promotion. In general, the promotion process requires a long time and mainly consists of the activation of the signalingsystem.

In many cancers, the function of cancer-related genes is regulated by the activation of various signaling systems through cytokines. Transforming growth factor-β (TGF-β) is involved in various functions such as cell growth inhibition, apoptosis, differentiation, and epithelial-mesenchymal transition (EMT) through the regulation of signaling pathways of SMAD, Rho, PP2A, and cancer-related genes. Many types of research have been conducted on various mechanisms and broad roles of TGF-β as a pleiotropic factor in animal models and patients.

The vestigial-like (VGLL) gene has structural similarity to the vestigial (vg) gene, a Drosophila transcription factor coactivator and acts as a cofactor for the transcription factors TEA domain transcription factors (TEADs) that bind to the TEA region. The functions and underlying mechanisms of VGLL family proteins in stomach cancer have been studied. VGLL3 overexpression in stomach cancer patients is known to be associated with a low survival rate. VGLL4, which binds TEAD competitively with Yes-associated protein (YAP), has been reported as a tumor suppressor. VGLL1 has high structural homology with YAP and TAZ (transcription coactivator with a PDZ-binding motif) to form the VGLL1-TEAD complex. VGLL1, which is highly expressed in stomach cancer and correlates with the prognosis of patients with PIK3CA or PIK3CB overexpression, has been reported as a gene associated with stomach cancer malignancy. In addition, it was revealed that the transcription of VGLL1 is regulated through the signaling mechanism of PI3K-AKT-β-catenin. In addition, it was confirmed that the VGLL1-TEAD complex was involved in the proliferation and metastasis of stomach cancer by increasing the expression of matrix metallopeptidase 9 (MMP9).

The specific action of VGLL1 in relation to the proliferation and metastasis of a series of cancer cells is unknown. With respect to VGLL1, a method for predicting the survival period of a patient with lung cancer using VGLL1 as a biomarker, or cancer cell proliferation or cancer metastasis related to the enhancement of VGLL1 expression has been reported in some documents.

Under this background, the present inventors have found that the peptide fragment of VGLL1 not only inhibits the expression of MMP9 but also suppresses the proliferation of cancer cells, thereby completing the present invention as a cancer therapeutic agent.

### [Disclosure]

### [Technical Problem]

An object of the present invention is to provide a VGLL1 peptide fragment or a variant thereof including serine at position 84 of the VGLL1 peptide sequence represented by SEQ ID NO: 1 and having a length of 35 amino acids or less.

Another object of the present invention is to provide a method for treating cancer including a step of administering the VGLL1 peptide fragment or a variant thereof to a subject.

### [Technical Solution]

One aspect of the present invention provides a VGLL1 peptide fragment or a variant thereof including serine at position 84 of the VGLL1 peptide sequence represented by SEQ ID NO: 1 and having a length of 35 amino acids or less.

According to one embodiment of the present invention, the VGLL1 peptide fragment or variant thereof may be a VGLL1 peptide fragment or variant thereof having a sequence of 6 to 25 amino acids.

According to one embodiment of the present invention, the VGLL1 peptide fragment or variant thereof may be a VGLL1 peptide fragment or a variant thereof including the serine sequence at position 84 in the amino acid sequence between positions 52 and 115 represented by SEQ ID NO: 1.

According to one embodiment of the present invention, the VGLL1 peptide fragment or variant thereof may be a VGLL1 peptide fragment or a variant thereof consisting of serine at position 84 and 6 to 20 amino acids adjacent thereto.

According to one embodiment of the present invention, the VGLL1 peptide fragment or variant thereof may be any one VGLL1 peptide fragment or variant thereof selected from the group consisting of SEQ ID NOs: 2 to 9.

According to one embodiment of the present invention, it may be a VGLL1 peptide fragment or a variant thereof further including a cell-penetrating peptide.

According to one embodiment of the present invention, the VGLL1 peptide fragment or variant thereof may include the cell-penetrating peptide which is represented by any one selected from the group consisting of SEQ ID NOs: 12 to 19.

According to one embodiment of the present invention, it may be a pharmaceutical composition for preventing or treating cancer including the VGLL1 peptide fragment or a variant thereof.

According to one embodiment of the present invention, it may be a pharmaceutical composition for preventing or treating cancer which is any one selected from the group consisting of stomach cancer, liver cancer, colorectal cancer, lung cancer, breast cancer, pancreatic cancer, squamous cell carcinoma of the head and neck, esophageal cancer, ovarian cancer, cervical cancer, endometrial cancer, mesothelioma, melanoma, sarcoma, osteosarcoma, liposarcoma, thyroid cancer, desmoid tumor, and hematologic malignancy.

According to one embodiment of the present invention, it may be a pharmaceutical composition for preventing or treating cancer which is stomach cancer.

Another aspect of the present invention provides a method for treating cancer including a step of administering the composition to a subject.

### [Advantageous Effects]

The peptide fragment of VGLL1 containing serine at position 84 of the present invention has an excellent effect in preventing and treating cancer by inhibiting the proliferation of cancer cells and suppressing the invasion of cancer cells.

### [Description of Drawings]

FIG. 1 shows the results confirming the increase in the expression of MMP9 by TGF-β treatment in cancer cells.
FIG. 2 shows the results of confirming the inhibition of proliferation and invasion of cancer cells caused by suppressing the expression of MMP9 in cancer cells.
FIG. 3 shows the results confirming the decrease in MMP9 promoter activity increased by TGF-β due to VGLL1 and TEAD4 inhibition in cancer cells through luciferase activity verification.
FIG. 4 shows the results confirming the decrease in MMP9 promoter activity increased by TGF-β due to VGLL1 and TEAD4 inhibition in cancer cells through RT-PCR.
FIG. 5 shows the results confirming that S84 of VGLL1 is related to the phosphorylation of VGLL1 by TGF-β.
FIG. 6 shows the results confirming that the serine at position 84 of VGLL1 is an important site for binding to TEAD4.
FIG. 7 shows the results confirming the effect of phosphorylation of VGLL1 on the increase in MMP9 expression by TGF-β.
FIG. 8 shows the results confirming that treatment with the peptide fragment including the serine at position 84 of VGLL1 reduces the increased MMP9 expression by the TGF-β treatment.
FIG. 9 shows the results confirming that cancer cell proliferation is inhibited by treatment with a peptide fragment containing the serine at position 84 of VGLL 1.
FIG. 10 shows the results confirming that the invasion of cancer cells is inhibited by treatment with a peptide fragment containing the serine at position 84 of VGLL 1.
FIG. 11 shows the results confirming that the proliferation of cancer cells is inhibited by treatment with the VGLL1 peptide fragment and a variant thereof.
FIG. 12 shows the results of confirming the inhibition of proliferation of various cancer cells by treatment with the S84 peptide fragment of VGLL1

### [Best Mode]

The present invention provides a VGLL1 peptide fragment or a variant thereof including serine at position 84 of the VGLL1 peptide sequence represented by SEQ ID NO: 1 and having a length of 35 amino acids or less.

Specifically, the variant of the VGLL1 peptide fragment including serine at position 84 of the VGLL1 peptide sequence represented by SEQ ID NO: 1 and having a length of 35 amino acids or less may be any sequence or a variant thereof including serine at position 84 of one represented by SEQ ID NO: 1 and having 35 amino acids or less between position 52 and position 115.

In the present invention, "peptide" and "protein" are used interchangeably and include reference to a polymer of amino acid residues. The terms apply to natural amino acid polymers as well as amino acid polymers in which one or more amino acid residues are artificial chemical analogs of the corresponding natural amino acid. The terms also apply to those polymers containing conservative amino acid substitutions such that the protein remains functional. In the present invention, the peptide may be synthesized using genetic recombination and a protein expression system and preferably synthesized *in vitro* through a peptide synthesizer or the like.

In the present invention, "VGLL1 peptide" refers to a polypeptide corresponding to the full-length VGLL1 protein. The term also includes any homolog, analog, fragment, or derivative of the VGLL1 protein. In one embodiment, the isolated VGLL1 peptide has the amino acid sequence (SEQ ID NO: 1). "VGLL1 encoding nucleic acid" means DNA or RNA encoding at least a portion of a VGLL1 polypeptide or variant thereof.

In the present invention, the VGLL1 peptide fragment having a length of 35 amino acids or less maybe 7 to 35 amino acids, preferably 7 to 25 amino acid residues, and more preferably 6 to 15 amino acids. It also includes an amino acid chain including a length of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 amino acids or any conceivable range therein.

The VGLL1 peptide fragment of the present disclosure, in some embodiments, may have no more than 35 contiguous amino acids in the amino acid sequence between positions 52 and 115 of the VGLL1 peptide sequence represented by SEQ ID NO: 1 and consist of any sequence including serine at position 84.

For example, when the VGLL1 peptide fragment according to the present invention may include the serine at position 84 of the sequence at the end, it may be a 30 amino acid sequence having the amino acid sequences between positions 55 and 84 of SEQ ID NO: 1. In addition, when the VGLL1 peptide fragment includes the 80th serine of the sequence at the end, it may be a 35 amino acid sequence having the amino acid sequences between positions 80 to 114 of SEQ ID NO: 1.

In addition, it may be any 35 or less sequences including the serine sequence at position 84 in the amino acid sequence between positions 52 and 115 of SEQ ID NO: 1. That is, amino acids having a sequence of 35 or less amino acids adjacent to and centering on the serine sequence at position 84 are the VGLL1 peptide fragments included in the category of the present invention.

For example, it may be any sequence such that the total number of amino acid sequences is 35 or less, centering on the serine sequence at position 84 represented by SEQ ID NO: 1. For example, it may be a sequence further including or consisting of serine at position 84 in the amino acid sequence of the VGLL1 protein and 8 to 25 amino acids, preferably 7 to 20 amino acids, more preferably 6 to 15 amino acids adjacent thereto.

More preferably, it may consist of 20 or less, 15 or less, or 10 or less, 9 or less, 8 or less, or 7 or less sequences centering on the sequence at position 84 represented by SEQ ID NO: 1.

Specifically, any sequence according to the present invention includes, for example, an amino acid sequence from any selected amino acid sequence at position 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, or 82 to any selected amino acid sequence at position 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, or 115 of the VGLL1 peptide sequence represented by SEQ ID NO:1. Any sequence may be included in the peptide fragment within the catagory of the present invention as long as the length satisfies the above-mentioned 35 or less.

More specifically, it may be the amino acid sequence represented by SEQ ID NO: 2 consisting of sequences between 77th to 91th of VGLL1. More specifically, it may be the amino acid sequence represented by SEQ ID NO: 9 consisting of sequences between 82th to 88th of VGLL1.

These exemplary VGLL1 peptide fragment sequences are shown in SEQ ID NOs: 2 to 9 of Table 1 below, and these sequences include the serine sequence at position 84 of the VGLL1 peptide, and correspond to the sequence shown as an example, and it is not limited to these exemplary sequences.

**[Table 1]**

| | | |
|---|---|---|
| SEQ ID NO: 2 | Sequence 77-91 | PNQWRYS SPWTKPQP |
| SEQ ID NO: 3 | Sequence 78-90 | NQWRYSSPWTKPQ |
| SEQ ID NO: 4 | Sequence 79-89 | QWRYSSPWTKP |
| SEQ ID NO: 5 | Sequence 80-88 | WRYSSPWTK |
| SEQ ID NO: 6 | Sequence 80-87 | WRYSSPWT |
| SEQ ID NO: 7 | Sequence 80-86 | WRYSSPW |
| SEQ ID NO: 8 | Sequence 81-87 | RYSSPWT |
| SEQ ID NO: 9 | Sequence 82-88 | YSSPWTK |

In the present invention, "biologically active" refers to a peptide fragment having a structural function, and/or a similar regulatory function, and/or a similar biochemical function similar to that of an individual wild-type polypeptide as well as a peptide according to the present invention.

In the present invention, a "variant" refers to any peptide (including a peptide encoded by a corresponding nucleic acid) having a substitution, deletion, or addition (or any combination thereof) from one (or more) amino acids or to a sequence, including allelic mutations, compared to a wild-type peptide. In some embodiments, the resulting polypeptide retains at least 75%, 80%, 85%, 90%, 95%, 99%, or more of biological activity as compared to a wild-type polypeptide when used in the present invention. Specifically, the variant according to the present invention is a sequence necessarily containing S84 of SEQ ID NO: 1 and is a sequence having 35 or less amino acids in which a mutation occurs at any site of the VGLL1 peptide between positions 52 and 115, maintaining biological activity as described above.

Sequence identity or homology can be measured using standard techniques known in the art. Alignment includes the introduction of gaps in the sequence being aligned. It is also understood that for sequences including more or less amino acids compared to the proteins disclosed herein, the percentage of homology is measured based on the number of homologous amino acids compared to the total number of amino acids. Consequently, variants of the VGLL1 polypeptide (including the polypeptide encoded by the corresponding nucleic acid) have at least 80%, at least 85%, at least 90%, at least 91%, at least 92%, at least 93%, at least 94%, at least 95%, at least 96%, at least 97%, at least 98%, or at least 99% sequence homology with the peptide sequence represented by SEQ ID NO: 1. Specifically, the variant according to the present invention is a variant of the VGLL1 peptide fragment including the serine at position 84 of the peptide sequence of VGLL1 represented by SEQ ID NO: 1 and having a length of 35 amino acids or less and has a mutation between positions 52 and 115 of SEQ ID NO: 1, having at least 80%, at least 85%, at least 90%, at least 91%, at least 92%, at least 93%, at least 94%, at least 95%, at least 96%, at least 97%, at least 98%, or at least 99% sequence homology, taking into account the corresponding sequence between positions 52 to 115 above.

In the present invention, a "deletion" is defined as a change in a nucleotide or amino acid sequence that lacks one or more nucleotide or amino acid residues, respectively, compared to a wild-type polynucleotide or polypeptide.

In the present invention, "insertion" or "addition" is a change in a nucleotide or amino acid sequence resulting from the addition of one or more nucleotide or amino acid residues, respectively, compared to a wild-type polynucleotide or polypeptide.

In the present invention, "substitution" results from the replacement of one or more nucleotides or amino acids by different nucleotides or amino acids, respectively, compared to a wild-type polynucleotide or polypeptide.

The cytokine TGF-b induces phosphorylation of intracellular signaling proteins. Treatment of TGF-b in cancer cells can induce phosphorylation of VGLL1. For example, a peptide fragment including S84 of VGLL1 can competitively inhibit the binding of VGLL1 and TEAD4 to suppress the expression of MMP9. Amino acids that can be phosphorylated by TGF-b include serine, threonine, and tyrosine. Accordingly, although not limited thereto, it may be preferable that the sequences at positions 54, 59, 61, 62, 64, 66, 74, 76, 82, 83, 84, and 96 in the amino acid sequence represented by SEQ ID NO: 1 are maintained without mutation.

Meanwhile, preferred amino acid mutation at the substitution position may be performed based on the similarity in polarity, charge, solubility, hydrophobicity, hydrophilicity, and/or amphiphilic properties of residues. For example, but not limited thereto, negatively charged amino acids may include aspartic acid and glutamic acid; positively charged amino acids may include lysine and arginine; and amino acids having an uncharged polar head group having a similar hydrophilicity value may include leucine, isoleucine, and valine; glycine and alanine; asparagine and glutamine; serine, threonine, phenylalanine, and tyrosine. Such mutations may include conservative substitutions. A "conservative substitution" refers to a substitution in which an amino acid is substituted with another amino acid having similar properties so that those skilled in the art can predict that the secondary structure and hydropathic nature (hydrophobic or hydrophilic natures) of the polypeptide are substantially unchanged. In general, the following groups of amino acids exhibit conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

For example, it may be exemplary amino acid substitutions that do not show a change in biological activity. It may, but be not limited thereto, include mutations at positions 85 and 87 of SEQ ID NO: 1. For example, it may be P85A or T85A mutation in SEQ ID NO: 1.

The present invention includes, but is not limited to, the following amino acid mutations at the aforementioned substitution positions: S-A, P->A, T->A, or Y->A.

Such variants may include non-conservative alterations. In a preferred embodiment, the variant polypeptide may differ from the native sequence by substitution, deletion, or addition of 5 amino acids or less. Variants may also be altered, for example, by deletion or addition of amino acids with minimal effect on the secondary structure and hydropathic natures of the polypeptide.

In some cases, the variant can also be modified by phosphorylation, sulfation, acrylation, glycosylation, methylation, farnesylation, acetylation, amidation, and the like.

Exemplary amino acid substituted variant sequences are shown in Table 2.

**[Table 2]**

| | | |
|---|---|---|
| SEQ ID NO: 10 | P85A | PNQWRYSSAWTKPQP |
| SEQ ID NO: 11 | T87A | PNQWRYSSPWAKPQP |

It contains the serine at position 84 of the VGLL1 peptide sequence represented by SEQ ID NO: 1, and the VGLL1 peptide fragment or variant thereof having a length of 35 amino acids or less may further include a cell-penetrating peptide sequence for intracellular penetration.

The cell-penetrating peptide is a kind of signal peptide and is a peptide sequence that aims to deliver substances into cells. It mainly consists of a peptide sequence of about 7 to 30 amino acids and may include any sequence having a signal peptide that can be delivered into a cell in the present invention.

For example, the cell-penetrating peptide mainly contains basic amino acid residues such as lysine/arginine, and thus plays a role in penetrating the fused proteins into the cell throught the cell membrane. The cell-penetrating peptide includes HIV-1 Tat protein, Drosophila antennapedia homeodomain (penetratin), HSV VP22 transcriptional regulatory protein, vFGF-derived MTS peptide, PTD-5, transpotan, or Pep-1 peptide-derived sequences, but are not limited thereto. As such, various CPPs identified/synthesized from viruses or cationic peptides (e.g., TAT48-60, penetratin (pAntp) 43-58, polyarginine, Pep-1, transportan, etc.) are biologically active substances and have an activity capable of cell internalization to mediate the movement of drug carriers.

Such a sequence may be linked to the N-terminus of the VGLL1 peptide fragment or variant thereof.

Exemplary sequences are shown in SEQ ID NOs: 12 to 19 below.

**[Table 3]**

| | |
|---|---|
| HIV-1 Tat (SEQ ID NO: 12) | YGRKKRRQRRR |
| Penetratin (SEQ ID NO: 13) | RQIKIWFQNRRMKWKK |
| HSV VP22 (SEQ ID NO: 14) | DAATATRGRSAASRPTERPRAPARSASR PRRPVE |
| MTS (SEQ ID NO: 15) | AAVALLPAVLLAAP |
| Transportan (SEQ ID NO: 16) | GWTLNSAGYLLGKINLKALAALAKKIL |
| PEP-1 (SEQ ID NO: 17) | KETWWETWWTEWSQPKKKRKV |
| Poly arginine (SEQ ID NO: 18) | RRRRRRR |
| PTD-5 (SEQ ID NO: 19) | RRQRRTSKLMKR |

Preferably, the sequence represented by SEQ ID NO: 12 may be linked to a VGLL1 peptide fragment or a variant thereof. Such a sequence may be, for example, a sequence peptide fragment such as SEQ ID NO: 27 or SEQ ID NO: 29. In addition, as shown in SEQ ID NO: 30 or SEQ ID NO: 31, a variant of a peptide fragment and a cell-penetrating sequence may be linked.

That is, the present invention may provide a fusion peptide including the cell-penetrating peptide; and a VGLL1 peptide fragment or a variant thereof including the serine at position 84 of the VGLL1 peptide sequence represented by SEQ ID NO: 1 and having a length of 35 amino acids or less.

The present invention also provides a nucleic acid sequence encoding the VGLL1 peptide fragment or variant thereof, and a fusion peptide in which a cell-penetrating peptide is linked thereto. "Nucleic acid" is intended to include DNA and RNA and may be double-stranded or single-stranded.

A nucleic acid encoding a peptide can be operably linked to a peptide produced in an appropriate expression system using expression vectors and methods well known in the art of molecular biology. A nucleic acid encoding a peptide disclosed herein can be incorporated into any expression vector that ensures the proper expression of the peptides. A possible expression vector is not limited so long as the vector is suitable for transformation of the host cell and includes plasmid, or modified virus (e.g., replication deficient retrovirus, adenovirus, and adeno-associated virus).

A recombinant expression vector "suitable for transformation of a host cell" is operatively linked to a nucleic acid molecule of the present disclosure and includes regulatory sequences selected based on the host cell used for expression. The terms "operatively linked" or "operably linked" are used interchangeably and are intended to mean that the nucleic acid is linked to regulatory sequences in a manner that enables expression of the nucleic acid.

Accordingly, the present disclosure provides a recombinant expression vector including a nucleic acid encoding a peptide and regulatory sequences necessary for transcription and translation of the inserted protein-sequence. Suitable regulatory sequences can be derived from a variety of sources, including bacterial, fungal, or viral genes. The selection of appropriate regulatory sequences is generally dependent on the selected host cell and can be readily accomplished by one of the ordinary skills in the art. Examples of such regulatory sequences include translation initiation signals and include transcriptional promoters and enhancers or RNA polymerase binding sequences, and ribosome binding sequences. In addition, depending on the host cell selected and the vector used, other sequences such as origins of replication, additional DNA restriction sites, enhancers, and sequences that confer transcriptional inducibility may be incorporated into the expression vector. It will also be understood that the necessary regulatory sequences may be supplied from the native protein and/or flanked regions thereof.

A recombinant expression vector can be introduced into a host cell to produce a transformed host cell. The term "transformed host cell" is intended to include prokaryotic and eukaryotic cells transformed or transfected with the recombinant expression vectors of the present disclosure. The terms "transformed with", "transfected with", "transformation", and "transfection" are intended to include introduction of a nucleic acid (e.g., a vector) into a cell by one of many possible techniques known in the art. Suitable host cells include a wide variety of prokaryotic and eukaryotic host cells. For example, the proteins of the present disclosure can be expressed in bacterial cells such as *E. coli,* insect cells (using baculovirus), yeast cells, or mammalian cells.

A nucleic acid molecule of the present disclosure may also be synthesized chemically, using standard techniques. A variety of methods are known for chemically synthesizing polydeoxy-nucleotides, including fully automated solid-phase synthesis in commercially available DNA synthesizers, such as peptide synthesis.

The present invention also provides a pharmaceutical composition including a VGLL1 peptide fragment or a variant thereof including the serine at position 84 of the VGLL1 peptide sequence represented by SEQ ID NO: 1 and having a length of 35 amino acids or less.

The present invention also provides a pharmaceutical composition for preventing or treating cancer including a VGLL1 peptide fragment or a variant thereof including the serine at position 84 of the VGLL1 peptide sequence represented by SEQ ID NO: 1 and having a length of 35 amino acids or less.

The present invention also provides a pharmaceutical composition for inhibiting cancer metastasis including a VGLL1 peptide fragment or a variant thereof including the serine at position 84 of the VGLL1 peptide sequence represented by SEQ ID NO: 1 and having a length of 35 amino acids or less.

The composition according to the present invention has excellent effects on the prevention, metastasis, and treatment of cancer because the peptide of VGLL1 inhibits cancer cell proliferation and inhibits cancer cell invasion. The composition according to the present invention includes both the direct therapeutic effect and the metastasis inhibitory effect as well as the action as an anticancer adjuvant.

In the present invention, "treatment" and "treating" refer to the administration or application of a therapeutic agent to a subject or performance of a procedure or modality in a subject for the purpose of obtaining the benefit of treatment of a disease or health-related condition. "Prevention," "preventing," and the like refer to an approach for preventing, inhibiting, or reducing the likelihood of occurrence or recurrence of a disease or condition. It also refers to delaying the onset or recurrence of a disease or condition or delaying the onset or recurrence of a disease or condition.

In the present invention, "subject" and "patient" refer to a human or non-human, such as a primate, mammal, or vertebrate. In certain embodiments, the subject is a human.

In the present invention, "therapeutic benefit" or "therapeutically effective" refers to anything that promotes or enhances the health of a subject in connection with the medical treatment of such a condition. This includes, but is not limited thereto, a decrease in the frequency or severity of signs or symptoms of a disease. For example, treating cancer may include reducing the size of the tumor, reducing the invasiveness of the tumor, reducing the rate of growth of cancer, or preventing metastasis. Treating cancer may also refer to prolonging the survival of a subject having cancer.

In the present invention, cancers include stomach cancer, liver cancer, colorectal cancer, lung cancer, breast cancer, pancreatic cancer, squamous cell carcinoma of the head and neck, esophageal cancer, ovarian cancer, cervical cancer, endometrial cancer, mesothelioma, melanoma, sarcoma, osteosarcoma, liposarcoma, thyroid cancer, desmoid tumor, and hematologic malignancy.

In the present invention, anticancer refers to negatively affecting cancer cells/tumors of a subject such as promoting the death of cancer cells, inducing apoptosis in cancer cells, reducing the growth rate of cancer cells, reducing the prevalence or number of metastases, reducing tumor size, inhibiting tumor growth, reducing blood supply to a tumor or cancer cells, promoting an immune response against cancer cells or tumors, preventing or inhibiting the progression of cancer, or increasing the lifespan of a subject with cancer.

In the present invention, the phrase "pharmaceutically or pharmacologically acceptable" refers to molecular substances and compositions that, when appropriate, do not cause side effects, allergic reactions, or other inappropriate reactions when administered to animals, such as humans. In light of the present disclosure, those skilled in the art will recognize the preparation of pharmaceutical compositions including antibodies or additional active components. It is also understood that, for animal (e.g., human) administration, manufacturing must meet sterility, pyrogenicity, general safety, and purity standards as required by the FDA Office of Biological Standards.

As mentioned above, the peptide fragment or variant thereof according to the present invention further includes a cell-penetrating peptide, so that it can be more easily delivered to cells in the human body.

The pharmaceutical composition to be used in the present invention may be formulated into a dosage form by means of a standard technique, using at least one physiologically acceptable carrier or excipient. A suitable pharmaceutical carrier is disclosed in the present invention and the document (Remington: The Science and Practice of Pharmacy, 21st Ed., University of the Sciences in Philadelphia, Lippencott Williams & Wilkins (2005)).

The peptides of the invention may be formulated to be administered via any suitable route, for example, an inhalation, local, nasal, oral, parenteral, or intrarectal route. Thus, the administration of the pharmaceutical composition mentioned above may be performed by means of an intradermal, subcutaneous, intravenous, intramuscular, intranasal, inhalational, intracerebral, endotracheal, intra-arterial, intraperitoneal, intravesical, intrapleural, intracoronary, subcutaneous or intratumoral injection, or by using a syringe or other devices. Percutaneous administration is also considered along with inhalation or aerosol administration. A tablet and capsule may be administered orally, rectally, or vaginally.

The pharmaceutical composition will include the polypeptide, which is conventionally dissolved in a pharmaceutically acceptable carrier, preferably an aqueous carrier, or the polypeptide. The peptide may be provided together or separately. Various aqueous carriers, for example, buffered salt water, etc., may be used. Such a solution has a bactericidal property and does not generally have an undesirable substance. Such composition may be sterilized by means of a conventional, widely known sterilization technique. The composition may contain a pharmaceutically acceptable adjuvant, as required to meet the physiological conditions, for example, a pH adjuster and buffer, toxicity adjusting agent, etc. for example, sodium acetate, sodium chloride, potassium chloride, calcium chloride, sodium lactate, etc. A concentration of the peptide in such dosage form may be extensively various, and may be selected mainly based on a fluid volume, viscosity, weight, etc. according to a selected certain administration mode and a patient's need.

The pharmaceutical composition of the present invention is suitable for a parenteral administration, including an intravenous or intracoelomic administration.

The peptide of the present invention may be formulated into a dosage form for the parenteral administration via an injection, for example, a bolus or continuous injection. The dosage form for injection may be present along with an added preservative in a unit-dosage form container, for example, an ampule or a multi-dose container. An injectable composition is preferably an aqueous isotonic solution or suspension, and a suppository is preferably prepared from a lipid emulsion or suspension. The composition may be sterilized and/or contain an adjuvant, for example, a preservative, stabilizer, humectant or emulsifier, dissolution promoter, osmoregulatory salt, and/or buffer. On the other hand, the active component may be present in a form of powder, which is made up before use by means of a suitable vehicle, for example, sterile pyrogen-free water. Also, the active component may contain other therapeutically valuable substances. The compositions are prepared according to a conventional mixing, granulation, or coating method, respectively, and contain about 0.1 to 75%, preferably about 1 to 50% of an active component.

In the case of an oral administration, the pharmaceutical composition or drug may take on a form of tablet or capsule, which is prepared, for example, by conventional means, along with a pharmaceutically acceptable excipient. It is preferable that such pharmaceutical composition or drug should be the tablet and gelatin capsule, containing the active component, that is, the composition of the present invention along with: (a) a diluent or filler, for example, lactose, dextrose, sucrose, mannitol, sorbitol, cellulose (for example, ethyl cellulose, and microcrystalline cellulose), glycine, pectin, polyacrylate and/or calcium hydrogen phosphate, and calcium sulphate; (b) a lubricant, for example, silica, talcum, stearic acid, magnesium or calcium salt thereof, metallic stearate, colloidal silicon dioxide, hydrogenated vegetable oil, maize starch, sodium benzoate, sodium acetate, and/or polyethylene glycol; also, in case of the tablet, (c) a binder, for example, magnesium aluminum silicate, starch paste, gelatin, tragacanth, methylcellulose, sodium carboxymethylcellulose, polyvinyl pyrrolidone, and/or hydroxypropyl methylcellulose; in some cases, (d) a disintegrating agent, for example, starch (for example, potato starch or sodium starch), glycollate, agar, alginic acid or sodium salt thereof, or an effervescent blend; (e) a humectant, for example, sodium lauryl sulphate; and/or (f) an absorbent, coloring agent, flavoring agent, and sweetening agent.

The present invention administers the pharmaceutical composition into a patient in a therapeutically effective dose for preventing, treating, or inhibiting a disease such as cancer, or a malignant condition thereof. The pharmaceutical composition is administered into the patient in a sufficient amount enough to draw an effective therapeutic or diagnostic response from the patient. The effective therapeutic or diagnostic response refers to the response, which at least partially inhibits or delays symptoms or complications of the disease or malignant condition. A suitable amount for performing such administration is defined as a "therapeutically effective amount."

A dosage of the peptide to be administered varies depending on a mammal's species, weight, age, individual condition, the surface area of a region to be treated, and administration type. The size of the dose may also be determined according to a presence, property, and degree of any side effect to a certain patient, which accompanies an administration of a certain compound.

A unit dosage to be administered into a mammal of about 50 to 80 kg, preferably a human, may contain the peptide in an amount of about 1 mg/kg to 5 mg/kg.

Typically, the dosage of the compound of the present invention is a sufficient dosage enough to achieve a targeted effect. An optimal administration schedule may be determined by measuring the peptide and calculating an accumulation thereof in the patient's body. Such composition may be provided at least once a day, week, month, or year. Those skilled in the art may easily determine an optimal dosage, administration method, and repetition rate. Those skilled in the art may determine an optimal administration for administering the peptide into humans according to an established protocol known in the art and disclosed in the present invention. However, it is to be understood that an actual dosage of an effective component should be determined considering various related factors such as a disease to be treated, a severity of the disease, an administration route, a patient's weight, age, gender, and the like, and thus the dosage is not construed to limit the scope of the present invention in any aspect.

The present invention also provides a VGLL1 peptide fragment or a variant thereof including the serine at position 84 of the VGLL1 peptide sequence represented by SEQ ID NO: 1 and having a length of 35 amino acids or less for use in the prevention or treatment of cancer.

The present invention also provides the use of a VGLL1 peptide fragment or a variant thereof including the serine at position 84 of the VGLL1 peptide sequence represented by SEQ ID NO: 1 and having a length of 35 amino acids or less in the preparation of a medicament for use in the prevention or treatment of cancer.

The present invention also provides a method of treating cancer including a step of administering a therapeutically effective amount of a VGLL1 peptide fragment or a variant thereof including the serine at position 84 of the VGLL1 peptide sequence represented by SEQ ID NO: 1 and having a length of 35 amino acids or less to a subject.

### [Modes of the Invention]

Hereinafter, preferred examples are presented to help the understanding of the present invention. However, the following examples are only provided for easier understanding of the present invention, and the content of the present invention is not limited by the examples.

In the present invention, it was confirmed that the VGLL1 peptide fragment or a variant thereof inhibits cancer cell proliferation and suppresses cancer cell invasion to have an excellent effect in preventing and treating cancer, thereby completing the present invention. Hereinafter, this will be described in detail.

### <Example 1> Confirmation of proliferation and metastasis of cancer cells by increased expression of MMP9 by TGF-β

### Example 1-1. Confirmation of expression of MMP9 by TGF-β

In order to confirm the expression change of MMP9 according to TGF-β treatment in stomach cancer cell lines, the human stomach cancer cell line NUGC3 was treated with TGF-β at each time period, then mRNA was extracted from the cells, and complementary DNA was synthesized. Then RT-PCR was performed using Matrix metalloproteinase 9 (MMP9) primers (Table 4).

**[Table 4]**

| Primer | Sequence |
|---|---|
| MMP9-F | 5'-TCTATGGTCCTCGCCCTGAA-3'(SEQ ID NO: 20) |
| MMP9-R | 5'-CATCGTCCACCGGACTCAAA-3'(SEQ ID NO: 21) |

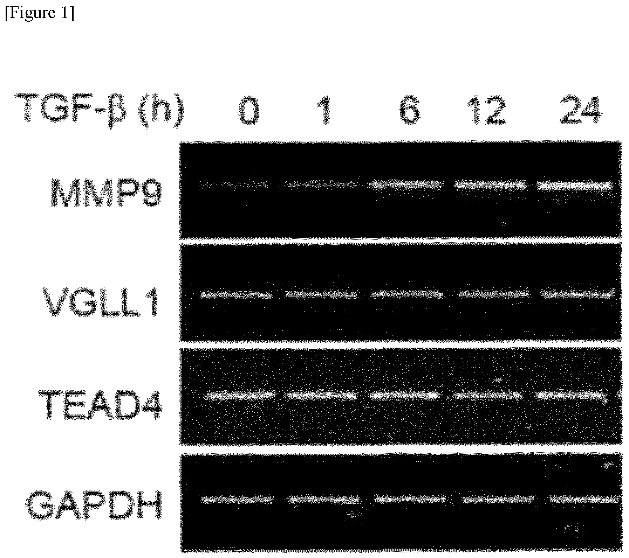

The results are shown in FIG. 1.

As shown in FIG. 1, it was confirmed that the expression of MMP9 was increased after 6 hours by TGF-β.

### Example 1-2. Confirmation of cancer cell proliferation and invasion by MMP9

In order to check the effect of MMP9 expression on the proliferation and metastasis of stomach cancer cells, the effect of siMMP9 treatment on the proliferation and invasion of cancer cells was confirmed.

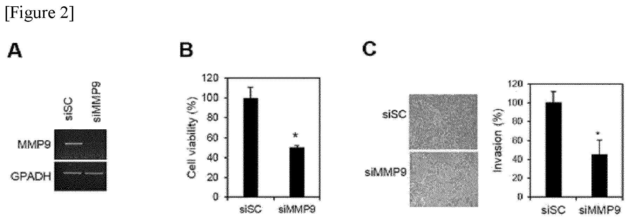

Specifically, the stomach cancer cells line NUGC3 was cultured in a 6-well plate overnight, and then treated with the mixture of Lipofectamine 2000 and 20 nM siSC or siMMP9 (SEQ ID NO: 22) and. After 48 hours, mRNA was extracted from the cells and complementary DNA was synthesized, followed by RT-PCR using the MMP9 primer (FIG. 2A).

**[Table 5]**

| | Sequence |
|---|---|
| siMMP9 | 5'-CCACAACAUCACCUAUUGGAUdTdT-3' |

In addition, in order to confirm the cell proliferation ability of MMP9, the stomach cancer cell line NUGC3 was cultured in a 96-well plate. The next day, cells were treated with the mixture of Lipofectamine 2000 and 20 nM siSC or siMMP9. After 48 hours, cells were fixed using 1% formalin solution. Thereafter, the cells were stained with 0.4% Sulforhodamine B solution for 1 hour, washed with acetic acid, and dried. Then, the cells were dissolved in a 10 mM Tris solution, and absorbance was measured at 540 nm using an ELISA plate reader (FIG. 2B).

Next, in order to confirm the cell invasive ability of MMP9, a cell culture insert was fixed in a 24-well plate. Matrigel was diluted 20-fold, and 100 µl of diluent was added. The reaction was carried out at 37°C for 1 hour or longer. 700 µl of medium containing 10% serum was added to a 24-well plate under the insert. MMP knockdown cells with the above siRNA were added to the insert according to 300 µl of serum-free medium to a concentration of 5 × 10⁵. After 48 hours, the insert was removed and cells were fixed in 10% formalin solution. Cells remaining inside the insert were removed with a cotton swab and washed with water. Cells were stained with 0.4% Sulforhodamine B solution for 1 hour, washed with acetic acid, and dried. Then, a photograph was taken with an optical microscope, the membrane on the insert was cut, and Sulforhodamine B in stained cells was dissolved in 10 mM Tris solution, and absorbance was measured at 540 nm using an ELISA plate reader (FIG. 2C).

The respective experimental results are shown in FIGS. 2A to 2C.

FIG. 2A shows the result of reduced expression of MMP9 by siRNA (siMMP9) treatment. In addition, FIG. 2B shows the greatly reduced cell viability by the reduced expression of MMP9 due to siRNA (siMMP9) treatment. In addition, FIG. 2C shows the greatly reduced cell invasiveness by siRNA (siMMP9) treatment.

As shown in the above results, it was confirmed that the MMP9 expression was inhibited by siMMP9 treatment, and thus the proliferation and invasion ability was greatly reduced in the stomach cancer cell line NUGC3.

### <Example 2> Confirmation of MMP9 promoter activity and action of VGLL1 and TEAD4 on mRNA expression thereof

### Example 2.1 Confirmation of MMP9 promoter activity by TGF-β and regulatory ability of VGLL1 and TEAD4

In order to determine whether VGLL1 and TEAD4 were involved in MMP9 promoter activity in NUGC3 human stomach cancer cell line, the MMP9 promoter luciferase activity was investigated after TGF-β treatment.

Specifically, the stomach cancer cell NUGC3 was cultured in a 48-well plate at a concentration of 2 × 10⁴ cells/well. The next day, the MMP9 gene-inserted pGL4.17-luciferase vector was transfected into cells using Lipofectamine 2000. After 24 hours, 20 nM siSC (SEQ ID NO: 23: 5'-CCUACGCCACCAAUUUCGUdTdT-3') siVGLL1 (SEQ ID NO 24: 5'-AGCCUAUAAAGACGGAAUGGAAUdTdT-3'), siTEAD4 (SEQ ID NO 25: 5'-GACACUACUCUUACCGCAUdTdT-3'), and siYAP (SEQ ID NO 26: 5 '-CCACCA AGCUAGAUAAAGAAAdTdT-3 ') were mixed with Lipofectamine 2000, and the cells were treated with the mixture. The next day, the cells were treated with TGF-β for 18 hours. Thereafter, the cells were disrupted using a passive lysis buffer. Then, after centrifuging cells, a supernatant was obtained. When the obtained supernatant was reacted with a substrate (product name: dual-luciferase assay kit), light was exhibited by a reaction between the luciferase enzyme and the substrate. At this time, the degree of luminescence of the cells was checked using Victor X Light.

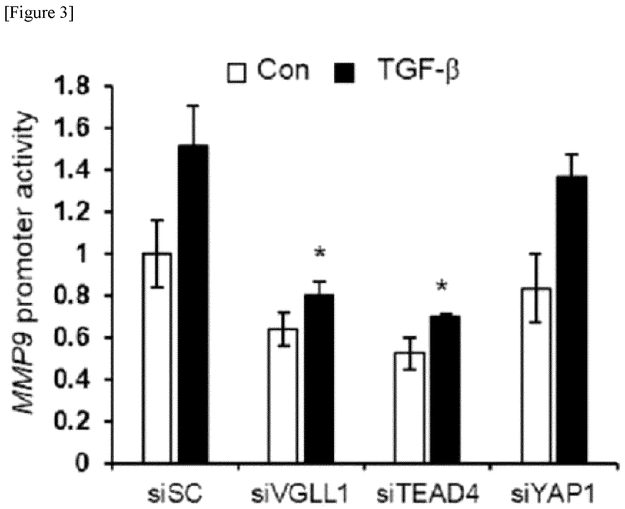

The results are shown in FIG. 3. As shown in FIG. 3, the MMP9 promoter activity increased by TGF-β was decreased in the cells transfected with siVGLL1 and siTEAD4 compared to the control (siSC). These results show that VGLL1 and TEAD4 regulate the promoter activity of MMP9.

### Example 2-2. Confirmation of the action of VGLL1 and TEAD4 on mRNA expression of MMP9 by TGF-β

In order to determine whether VGLL1 and TEAD4 were involved in MMP9 mRNA expression in NUGC3 human stomach cancer cells, MMP9 mRNA expression was examined by RT-PCR after TGF-β treatment.

Specifically, the stomach cancer cell line NUGC3 was cultured in a 6-well plate. The next day, 20 nM siSC, siVGLL1, siTEAD4, and siYAP were mixed with Lipofectamine 2000, and the cell line was treated with the mixture. The next day, after treatment with TGF-β for 18 hours, mRNA was extracted from the cells and complementary DNA was synthesized, followed by RT-PCR.

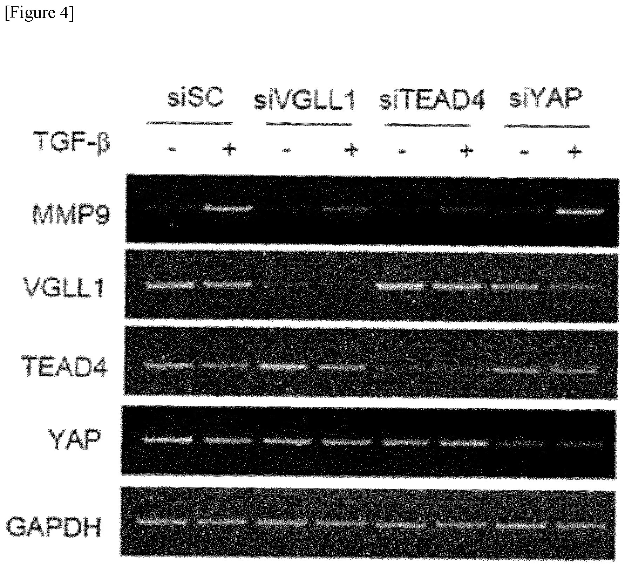

The results are shown in FIG. 4. As shown in FIG. 4, the MMP9 mRNA expression increased by TGF-β was decreased in the cells transfected with siVGLL1 and siTEAD4 compared to the control (siSC).

### <Example 3> Confirmation of phosphorylation of serine at position 84 of VGLL1 by TGF-β

In order to check whether VGLL1 was phosphorylated by TGF-β, serines at positions 50, 84, and 124 of the VGLL1 amino acid sequence were mutated to alanine, and then, phosphorylation of VGLL 1 was examined.

Specifically, serines at positions 50, 84, and 124 of VGLL1 were replaced with alanine using site-direct mutagenesis system in pcDNA3-HA-VGLL1-vector.

In addition, the stomach cancer cell line NUGC3 was cultured in a 6-well plate. The next day, pcDNA3-HA (EV), pcDNA3-HA-VGLL1 (WT), pcDNA3-HA-VGLL1-S50A (S50A mutation), pcDNA3-HA-VGLL1-S84A (S84A mutation), and pcDNA3-HA-VGLL1-S124A (S124A mutation) were mixed with Turbofect, and the cells were transformed with the mixture. After 48 hours, proteins were extracted from the cells treated with TGF-β for 15 minutes using RIPA buffer. The extracted protein was subjected to immunoprecipitation using agarose bound with HA antibody and separated by electrophoresis. Thereafter, western blot analysis was performed using antibodies on the polyvinylidene fluoride membrane.

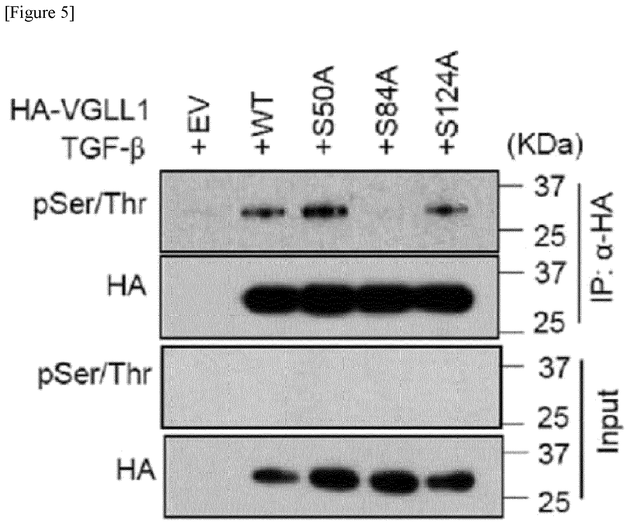

The results are shown in FIG. 5. As can be seen in FIG. 5, phosphorylation of VGLL1 by TGF-β was detected by serine/threonine phosphorylation (pSer/Thr) antibodies in WT, S50A, and S124A. On the other hand, VGLL1 phosphorylation was not detected in the cells expressing the S84A mutation. This is a result showing that the S84 of VGLL1 is phosphorylated by TGF-β signaling.

### <Example 4> Confirmation of phosphorylated VGLL1 and TEAD4 binding

In order to confirm whether VGLL1 binds to the transcription factor TEAD4, immunoprecipitation was performed. In order to verify whether VGLL1 phosphorylation was important, the vector used in <Example 3> was used.

Specifically, the GFP-TEAD4 vector and pcDNA3-HA (EV), pcDNA3-HA-VGLL1 (WT), pcDNA3-HA-VGLL1-S50A (S50A mutation), pcDNA3-HA-VGLL1-S84A (S84A mutation), and pcDNA3-HA-VGLL1-S124A (S124A mutation) were mixed with Turbofect. The mixture was transformed to the cell lines cultured for 24 hours. After 48 hours, the protein was extracted from the cells using a Ripa buffer. The extracted protein was subjected to immunoprecipitation using agarose bound with HA antibody and separated by electrophoresis. Thereafter, western blot analysis was performed using an antibody on the polyvinylidene fluoride membrane.

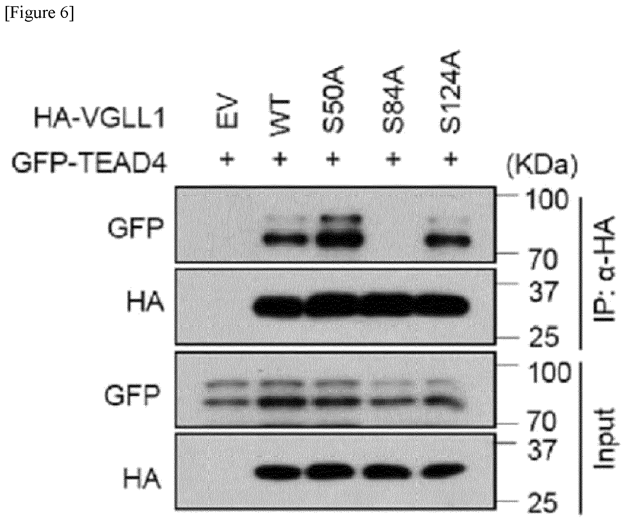

The results are shown in FIG. 6. As shwon in FIG. 6, VGLL1 and TEAD4 binding was detected by GFP antibodies in WT, S50A, and S124A. On the other hand, TEAD4 binding was not be detected in the cells expressing S84A mutation. This result showed that S84 of VGLL1 corresponds to an important site for binding to TEAD4.

### <Example 5> The effect of VGLL1 phosphorylation to increase MMP9 expression by TGF-β

In order to confirm whether VGLL1 phosphorylation was involved in MMP9 protein expression in the NUGC3 human stomach cancer cells, MMP9 protein expression was examined by western blot analysis using the VGLL1 vectors used in <Example 3>.

Specifically, the stomach cancer cell line NUGC3 was cultured in a 6-well plate. The next day, each vector, pcDNA3-HA (EV), pcDNA3-HA-VGLL1 (WT), pcDNA3-HA-VGLL1-S50A (S50A), pcDNA3-HA-VGLL1-S84A (S84A), and pcDNA3-HA-VGLL1-S124A (S124A), was mixed with Turbofect and placed in NUGC3 cells. After 24 hours, TGF-β was treated for 18 hours, and proteins were extracted from the cells using a Ripa buffer. The extracted protein was separated by electrophoresis. Thereafter, western blot analysis was performed using MMP9, HA, and GAPDH antibodies.

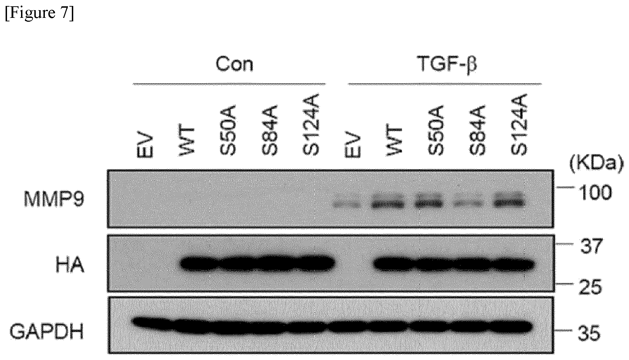

The results are shown in FIG. 7. As shown in FIG. 7, the MMP9 expression by TGF-β was decreased in the cells in which serine was substituted with alanine at position 84 of VGLL1. This indicates that VGLL1 phosphorylation by TGF-β is important for MMP9 expression and that serine at position 84 of the VGLL1 peptide is an important factor.

### <Example 6> The effects of S84 VGLL1 peptide fragment on the inhibition of MMP9 expression and cancer cell proliferation and invasion

### Example 6-1. S84 VGLL1 peptide reduced MMP9 expression induced by TGF-β

To evaluate whether the peptide fragment that interferes the binding between VGLL1 and TEAD4 can inhibit MMP9 expression in human stomach cancer cells, peptides shown in Table 6 were prepared to examine their effects on MMP9 expression.

**[Table 6]**

| Peptide fragment | Sequence |
|---|---|
| VGLL1 (S84) | YGRKKRRQRRRPNQWRYSSPWTKPQP (SEQ ID NO: 27) |
| VGLL1 (S84A) | YGRKKRRQRRRPNQWRYSAPWTKPQP (SEQ ID NO: 28) |
| Control peptide | YGRKKRRQRRR (SEQ ID NO: 12) |

The peptide fragment sequence in Table 6 provides the 77th to 91st sequences of VGLL1 including S84 of VGLL1. Specifically, the sequence represented by SEQ ID NO: 27 includes a fluorescein moiety (FITC) and YGRKKRRQRRR, which is an HIV TAT transduction domain sequence required for introduction into cells, and thus, it is linked to the 77th to 91st sequences of VGLL1. SEQ ID NO: 28 shows a sequence in which S84 of VGLL1 is substituted with S84A. SEQ ID NO: 12 represents the HIV TAT transduction domain sequence as a control sequence.

Experiments were performed using the synthetic peptide fragments. The stomach cancer NUGC3 cells were cultured in a 6-well plate. The next day, cells were cultured in serum-free cell culture for 24 hours. TGF-β and a control peptide fragment (SEQ ID NO: 12), a peptide fragment containing serine at position 84 of VGLL1 (SEQ ID NO: 27), and a mutant peptide fragment containing alanine at position 84 of VGLL1 (SEQ ID NO: 28) were treated into cells for 18 hours. Proteins were extracted from the cells using a Ripa buffer and were separated by electrophoresis. Thereafter, protein expression of genes was confirmed by using an antibody on the polyvinylidene fluoride membrane.

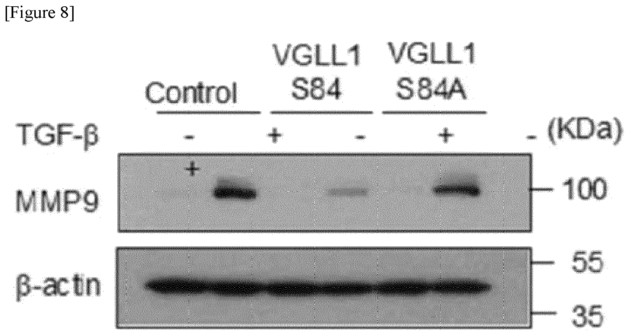

The results are shown in FIG. 8. As can be seen in FIG. 8, the increased MMP9 expression by TGF-β was reduced by treatment with a peptide fragment containing serine at position 84 of VGLL1. On the other hand, cells were not affected by the treatment with the mutant peptide fragment sequence containing alanine at position 84 of VGLL1. From these results, it was confirmed that the peptide fragment containing serine at position 84 of VGLL1 according to the present invention can exhibit an excellent effect in the treatment of cancer.

### Example 6-2. Confirmation of cancer cell proliferation inhibitory effect by S84 VGLL1 peptide fragment

The reduction of MMP9 expression by interfering with the binding between VGLL1 and TEAD4 in stomach cancer cells can also affect the proliferation of cancer cells. Thus, it was evaluated whether VGLL1 peptide fragment including S84 inhibited cell proliferation.

Specifically, the stomach cancer cell line NUGC3 was cultured in a 96-well plate. The next day, the cells were treated with the peptide fragment. The cell proliferation was analyzed using a real-time cell analysis system (product name: IncuCyte Zoom).

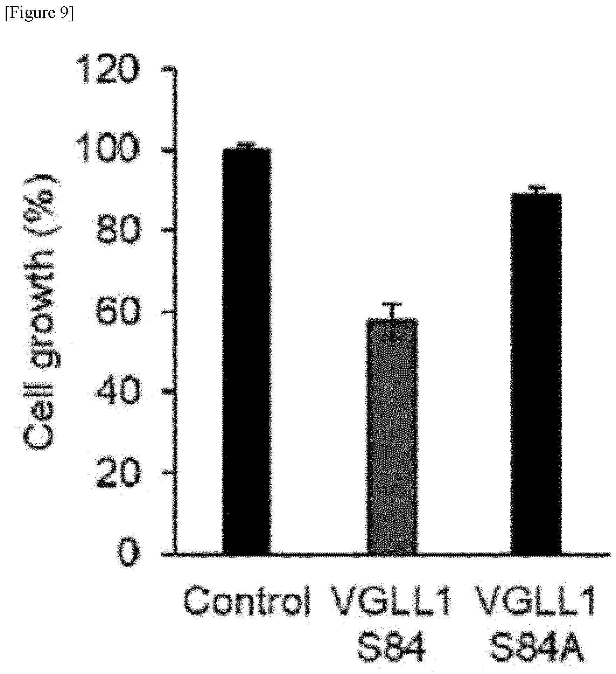

The results are shown in FIG. 9. As can be seen in FIG. 9, the S84 VGLL1 peptide fragment exhibited an effect of inhibiting cancer cell proliferation. On the other hand, the control peptide fragment or the mutated VGLL1 peptide fragment containing an alanine at position 84 in which the position of S84 was mutated did not have any effect on the inhibition of cancer cell proliferation.

From the above results, it was confirmed that the S84 VGLL1 peptide fragment sequence according to the present invention exhibits an excellent effect in inhibiting cancer cell proliferation.

### <Example 7> Confirmation of inhibition of cancer cell invasion by S84 VGLL1 peptide fragment

A method similar to Example 1-2 was performed for stomach cancer cell invasion experiment by the VGLL1 peptide fragment.

The stomach cancer cell line NUGC3 was placed in an insert together with the peptide fragment. After 48 hours of cell culture, the insert was fixed in a 10% formalin solution, and the downwardly migrated cells were confirmed by staining with 0.4% Sulforhodamine B solution.

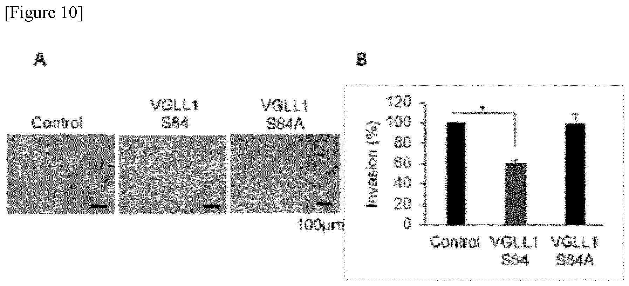

The results are shown in FIG. 10. As can be seen in FIG. 10, the VGLL1 peptide fragment sequence including S84 exhibited an effect of inhibiting cancer cell invasion, whereas the sequence in which the corresponding position was mutated did not exhibit such an effect.

### <Example 8> Confirmation of cancer cell proliferation inhibitory effect of variant of S84 VGLL1 peptide fragment

In order to confirm the cell proliferation inhibitory effect by mutation of major amino acids among the VGLL1 S84 peptide sequences, peptides were prepared as shown in Table 7.

**[Table 7]**

| Peptide | Sequence |
|---|---|
| Control | YGRKKRRQRRR (SEQ ID NO: 12) |
| VGLL1 S84-S | YGRKKRRQRRR-YSSPWTK (SEQ ID NO: 29) |
| VGLL1 S84 | YGRKKRRQRRR-PNQWRYSSPWTKPQP (SEQ ID NO: 27) |
| VGLL1 S84A | YGRKKRRQRRR-PNQWRYSAPWTKPQP (SEQ ID NO: 28) |
| VGLL1 P85A | YGRKKRRQRRR-PNQWRYSSAWTKPQP (SEQ ID NO: 30) |
| VGLL1 T87A | YGRKKRRQRRR-PNQWRYSSPWAKPQP (SEQ ID NO: 31) |

The stomach cancer cell line NUGC3 was cultured in a 96-well plate, and the next day, the cells were treated with 60 µM peptide. The cell proliferation was analyzed using a real-time cell analysis system (product name: Incucyte).

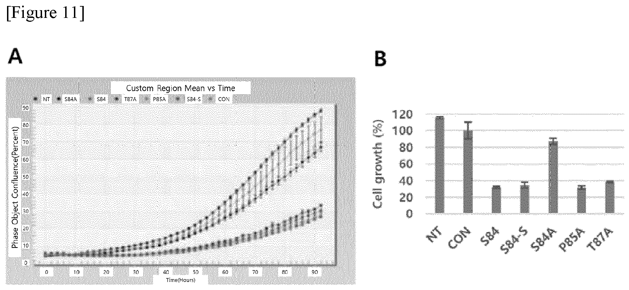

The results are shown in FIG. 11. FIG. 11A is a graph of real-time cell proliferation, and FIG. 11B is a graph showing cell proliferation after 4 days based on 100% control peptide.

As can be seen from the figures, cells were treated with the S84 VGLL1 peptide as well as S84-S, P85A, T87A, etc. to show an excellent effect in inhibiting cancer cell proliferation.

Based on these results, it was confirmed that the S84 sequence was the most important as the sequence of the peptide showing the cell proliferation inhibitory effect.

From the above results, it was confirmed that the peptide fragment sequence containing the S84 serine of VGLL1 according to the present invention exhibits an excellent effect in the treatment of cancer through inhibition of proliferation and invasion of cancer cells.

### <Example 9> Confirmation of various cancer cell proliferation inhibitory effects of S84 VGLL1 peptide fragment

In order to confirm the various cancer cell proliferation inhibitory effects of the variant of S84 VGLL1 peptide fragment, the VGLL1 S84 peptide sequence represented by SEQ ID NO: 27 was treated into various cancer cell lines.

Specifically, the cancer cells of Table 8 were cultured in a 96-well plate and treated with 60 µM peptide on the next day, and cell proliferation was analyzed using a real-time cell analysis system (product name: IncuCyte Zoom).

**[Table 8]**

| Type of cancer | Name of cancer cell line |
|---|---|
| Stomach cancer | NUGC3 |
| | AG5 |
| | NCI-N87 |
| | SNU484 |
| | MKN1 |
| Colorectal cancer | SW620 |
| | HCT116 |
| | WiDr |
| Lung cancer | A549 |
| | H1650 |
| | H1299 |
| Liver cancer | HepG2 |
| | SNU354 |
| Pancreatic cancer | ASPC1 |
| | PANC 1 |
| | Mia-paca2 |
| Breast cancer | MCF7 |
| | SKBR3 |
| Cervical cancer | Hela |
| Ovarian cancer | SK-OV3 |

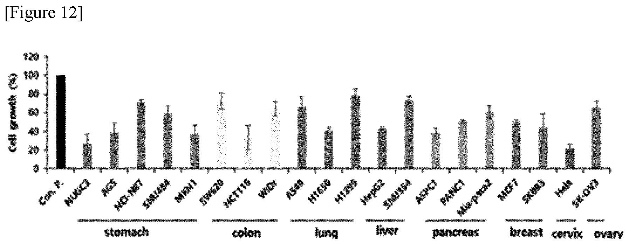

As a result, as can be seen in FIG. 12, it was confirmed that the S84 VGLL1 peptide fragment exhibited an excellent effect in inhibiting the proliferation of not only stomach cancer cells, but also colorectal cancer, lung cancer, liver cancer, prostate cancer, breast cancer, cervical cancer, and ovarian cancer cells.

Based on these results, it was confirmed that the S84 sequence was important as a sequence of peptides showing the effect of inhibiting various cancer cell proliferation.

As above, a specific part of the present invention has been described in detail. For those of ordinary skill in the related art, this specific description is only a preferred embodiment, and it is clear that the scope of the present invention is not limited thereto. Accordingly, the substantial scope of the invention is defined by the appended claims and their equivalents.

## Claims

1. A VGLL1 peptide fragment or variant thereof comprising serine at position 84 of a VGLL1 peptide sequence represented by SEQ ID NO: 1 and having a length of 35 amino acids or less.

2. The VGLL1 peptide fragment or variant thereof of claim 1, wherein the VGLL1 peptide fragment or variant thereof has a sequence of 6 to 25 amino acids.

3. The VGLL1 peptide fragment or variant thereof of claim 1, wherein the VGLL1 peptide fragment or variant thereof includes serine at position 84 in the amino acid sequence between positions 52 and 115 represented by SEQ ID NO: 1.

4. The VGLL1 peptide fragment or variant thereof of claim 1, wherein the VGLL1 peptide fragment or variant thereof consists of the serine at the position 84 and 6 to 20 amino acids adjacent thereto.

5. The VGLL1 peptide fragment or variant thereof of claim 1, wherein the VGLL1 peptide fragment or variant thereof is represented by any one selected from the group consisting of SEQ ID NOs: 2 to 9.

6. The VGLL1 peptide fragment or variant thereof of claim 1, further comprising a cell-penetrating peptide.

7. The VGLL1 peptide fragment or variant thereof of claim 6, wherein the cell-penetrating peptide is represented by any one selected from the group consisting of SEQ ID NOs: 12 to 19.

8. A pharmaceutical composition for preventing or treating cancer, the composition comprising the VGLL1 peptide fragment or the variant thereof of any one of claims 1 to 7.

9. The pharmaceutical composition of claim 8, wherein the cancer is any one selected from the group consisting of stomach cancer, liver cancer, colorectal cancer, lung cancer, breast cancer, pancreatic cancer, squamous cell carcinoma of the head and neck, esophageal cancer, ovarian cancer, cervical cancer, endometrial cancer, mesothelioma, melanoma, sarcoma, osteosarcoma, liposarcoma, thyroid cancer, desmoid tumor, and hematologic malignancy.

10. The pharmaceutical composition of claim 9, wherein the cancer is stomach cancer.

11. A method of treating cancer, the method comprising a step of administering the composition of claim 1 to a subject.
